# EUROPÄISCHE PATENTANMELDUNG

(11) **EP 1 191 816 A2**
(43) Veröffentlichungstag der Anmeldung: **27.03.2002**
(21) Anmeldenummer: 01118055.1
(22) Anmeldetag: 25.07.2001
(51) Int. Cl.: H04R 25/00

(54) **Mindestens teilweise implantierbares Hörsystem**

(30) Priorität: 25.09.2000 DE 10047388
(71) Anmelder: Cochlear Limited, Lane Cove, NSW 2066 (AU)
(72) Erfinder: Müller, Gerd M., Dr.rer.nat. Dipl.-Phys., 85716 Lohhof (DE); Leysieffer, Hans, Dr.-Ing., 82024 Taufkirchen (DE)
(74) Vertreter: Schwan - Schwan - Schorer

(57) **Zusammenfassung**

Mindestens teilweise implantierbares Hörsystem mit mindestens einem ausgangsseitigen elektromechanischen Wandler (12) und einem an der Schädelkalotte mittels einer Befestigungsanordnung (19) fest montierbaren Mikromanipulator (18) zum Positionieren des Wandlers und zum Fixieren des Wandlers in einer mittels des Mikromanipulators eingestellten Position. Zwischen dem Wandler (12) und dem Mikromanipulator (18) befindet sich eine lösbare Kupplung (21, 45, 50, 60, 62, 68; 22, 54, 58), die in einem montierten Zustand den Wandler mit Bezug auf den Mikromanipulator festlegt und die bei Lösen ein Abnehmen des Wandlers von dem Mikromanipulator erlaubt.

## Beschreibung

Die vorliegende Erfindung betrifft ein mindestens teilweise implantierbares Hörsystem mit mindestens einem ausgangsseitigen elektromechanischen Wandler und einem an der Schädelkalotte mittels einer Befestigungsanordnung fest montierbaren Mikromanipulator zum Positionieren des Wandlers und zum Fixieren des Wandlers in einer mittels des Mikromanipulators eingestellten Position.

Unter mindestens teilweise implantierbaren Hörsystemen sollen vorliegend Systeme verstanden werden, bei denen das Schallsignal mit mindestens einem Sensor, der ein Schallsignal in ein elektrisches Signal umwandelt (Mikrofonfunktion), aufgenommen und elektronisch weiterverarbeitet und verstärkt wird und deren ausgangsseitiges Signal das geschädigte Gehör auf elektromechanische Weise stimuliert, wobei mindestens eine Komponente des Systems, insbesondere ein ausgangsseitiger elektromechanischer Wandler, zur Implantation ausgebildet ist.

Unter dem Begriff "Hörstörung" sollen vorliegend alle Arten von Innenohr- und Mittelohrschäden sowie deren beliebige Kombinationen sowie auch zeitweise auftretende oder permanente Ohrgeräusche (Tinnitus) verstanden werden.

Hörsysteme der vorliegend betrachteten Art umfassen allgemein mindestens einen schallaufnehmenden Sensor (Mikrofon), eine elektronische Anordnung zur Audiosignalverarbeitung und -verstärkung, eine elektrische Energieversorgungseinheit, welche einzelne Komponenten des Systems mit Strom versorgt, eine drahtlose, transkutane Ladevorrichtung im Falle eines Vollimplantates bei Verwendung eines implantatseitig sekundären elektrischen Speicherelements, sowie eine ausgangsseitige elektromechanisch aktorische Anordnung, die aus mindestens einem elektromechanischen Wandler zur Stimulation des Mittel und/oder Innenohres besteht, der mit einem vorliegend als Mikromanipulator bezeichneten mechanischen Positionier- und Fixationssystem verbunden ist, das fest und dauerhaft an der Schädelkalotte fixiert ist.

Elektronische Maßnahmen zur Rehabilitation eines operativ nicht behebbaren Innenohrschadens haben heute einen wichtigen Stellenwert erreicht. Bei totalem Ausfall des Innenohres sind Cochlea Implantate mit direkter elektrischer Reizung des verbleibenden Hörnerven im routinemäßigen klinischen Einsatz. Bei mittleren bis schweren Innenohrschäden kommen derzeit erstmals volldigitale Hörgeräte zur Anwendung, die eine neue Welt der elektronischen Audiosignalverarbeitung eröffnen und erweiterte Möglichkeiten der gezielten audiologischen Feinanpassung der Hörgeräte an den individuellen Innenohrschaden bieten. Trotz dieser in den letzten Jahren erreichten, erheblichen Verbesserungen der apparativen Hörgeräteversorgung bleiben bei konventionellen Hörgeräten grundsätzliche Nachteile bestehen, die durch das Prinzip der akustischen Verstärkung bedingt sind, das heißt insbesondere durch die Rückwandlung des elektronisch verstärkten Signals in Luftschall. Zu diesen Nachteilen zählen Aspekte wie die Sichtbarkeit der Hörgeräte, mangelnde Klangqualität aufgrund der elektromagnetischen Wandler (Lautsprecher), verschlossener äußerer Gehörgang sowie Rückkoplungseffekte bei hoher akustischer Verstärkung.

Aufgrund dieser prinzipiellen Nachteile besteht seit langem der Wunsch, von konventionellen Hörgeräten mit akustischer Anregung des geschädigten Innenohres abzuweichen und diese durch teil- oder vollimplantierbare Hörsysteme mit einer direkten mechanischen Stimulation zu ersetzen. Implantierbare Hörsysteme unterscheiden sich von konventionellen Hörgeräten: zwar wird das Schallsignal mit einem adäquaten Mikrofon in ein elektrisches Signal umgewandelt und in einer elektronischen Signalverarbeitungsstufe verstärkt; dieses verstärkte elektrische Signal wird jedoch nicht einem elektroakustischen Wandler (Lautsprecher) zugeführt, sondern einem implantierten elektromechanischen Wandler, dessen ausgangsseitigen mechanischen Schwingungen unmittelbar, also mit direktem mechanischen Kontakt dem Mittel- beziehungsweise Innenohr zugeführt werden oder mittelbar durch einen Kraftschluss über einen Luftspalt bei zum Beispiel elektromagnetischen Wandlersystemen. Dieses Prinzip gilt unabhängig von einer teilweisen oder vollständigen Implantation aller notwendigen Systemelemente sowie auch unabhängig davon, ob eine reine Innenohrschwerhörigkeit bei vollständig intaktem Mittelohr oder eine kombinierte Schwerhörigkeit (Mittel- und Innenohr geschädigt) rehabilitiert werden soll. Daher sind in der jüngeren wissenschaftlichen Literatur sowie in zahlreichen Patentschriften implantierbare elektromechanische Wandler sowie Verfahren zur Ankopplung der mechanischen Wandlerschwingungen an das intakte Mittelohr beziehungsweise das Innenohr direkt zur Rehabilitation einer reinen Innenohrschwerhörigkeit sowie auch an verbleibende Ossikel des Mittelohres bei artifiziell oder pathologisch verändertem Mittelohr zur Versorgung einer Schalleitungsschwerhörigkeit sowie deren Kombinationen beschrieben worden.

Als elektromechanische Wandlerverfahren kommen grundsätzlich alle physikalischen Wandlungsprinzipien in Frage wie elektromagnetisch, elektrodynamisch, magnetostriktiv, dielektrisch und piezoelektrisch. Verschiedene Forschungsgruppen haben sich in den letzten Jahren im wesentlichen auf zwei dieser Verfahren konzentriert: elektromagnetisch und piezoelektrisch. Eine Übersicht über diese Wandlervarianten findet sich bei ZENNER und LEYSIEFFER (HNO 1997 Vol. 45, S. 749-774).

Beim piezoelektrischen Verfahren ist eine mechanisch direkte Kopplung der ausgangsseitigen Wandlerschwingungen an die Mittelohrossikel oder direkt an das ovale Fenster notwendig; beim elektromagnetischen Prinzip kann die Kraftkopplung einerseits über einen Luftspalt erfolgen ("kontaktlos"), das heißt, nur der Permanentmagnet wird durch dauerhafte Fixation in direkten mechanischen Kontakt mit einem Mittelohrossikel gebracht. Andererseits besteht die Möglichkeit, den Wandler vollständig in einem Gehäuse zu realisieren (Spule und Magnet sind mit kleinstmöglichem Luftspalt gekoppelt) und die ausgangsseitigen Schwingungen über ein mechanisch steifes Koppelelement mit direktem Kontakt auf die Mittelohrossikel zu übertragen (LEYSIEFFER et al. 1997 (HNO 1997, Vol. 45, pp. 792-800),

Bei den bekannten Wandler- und Ankopplungsvarianten sind grundsätzlich zwei Implantationsprinzipien zu unterscheiden:
a) Einerseits befindet sich der elektromechanische Wandler mit seinem aktiven Wandlerelement selbst im Mittelohrbereich in der Paukenhöhle, und er ist dort mit einem Ossikel oder dem Innenohr direkt verbunden (zum Beispiel US-A-5 707 338).
b) Andererseits befindet sich der elektromechanische Wandler mit seinem aktiven Wandlerelement außerhalb des Mittelohrbereiches in einer artifiziell geschaffenen Mastoidhöhle; die ausgangsseitigen mechanischen Schwingungen werden dann mittels mechanisch passiver Koppelelemente über geeignete operative Zugänge (natürlicher aditus ad antrum (US-A-6 077 215), Eröffnung des chorda-facialis-Winkels oder über eine artifizielle Bohrung vom Mastoid aus)) zum Mittel- beziehungsweise Innenohr übertragen (US-A-5 277 694, DE-C-198 40 211). Bei dieser Zugangsart ist ein implantierbares Positionier- und Fixationssystem (Mikromanipulator) zur "Aufhängung" des elektromechanischen Wandlers notwendig, das mechanisch fest und dauerhaft mit dem Schädel verbunden sein muss (US-A-5 788 711 und DE-A-199 15 684).

Ein Vorteil der Varianten nach a) besteht darin, dass der Wandler als sogenannter "Floating Mass"-Wandler ausgeführt sein kann, das heißt, das Wandlerelement benötig keine "Reaktio" über eine feste Verschraubung mit dem Schädelknochen, sondern es schwingt aufgrund von Massenträgheitsgesetzen mit seinem Wandlergehäuse und überträgt diese Schwingungen direkt auf ein Mittelohrossikel (zum Beispiel die vorstehend erwähnte US-A-5 707 338). Dies bedeutet einerseits, dass vorteilhaft auf ein implantierbares Fixationssystem an der Schädelkalotte verzichtet werden kann; andererseits bedeutet diese Variante nachteilig, dass voluminöse artifizielle Element in die Paukenhöhle eingebracht werden müssen und deren Langzeit- und Biostabilität insbesondere bei temporären pathologischen Veränderungen des Mittelohres (zum Beispiel otits media) heute nicht bekannt beziehungsweise gewährleistet sind. Ein weiterer wesentlicher Nachteil besteht darin, dass die Wandler vom Mastoid aus mit ihrer elektrischen Zuleitung ins Mittelohr gebracht werden und dort mit Hilfe geeigneter operativer Werkzeuge fixiert werden müssen; dies erfordert einen erweiterten Zugang durch den chorda-facialis-Winkel und bringt somit eine latente Gefährdung des in unmittelbarer Nachbarschaft gelegenen Gesichtsnerven (nervus facialis) mit sich. Weiterhin sind solche "Floating-Mass-Wandler" dann nur noch sehr eingeschränkt oder überhaupt nicht mehr einsetzbar, wenn das Innenohr zum Beispiel über das ovale Fenster direkt stimuliert werden soll, weil aufgrund pathologischer Veränderungen zum Beispiel der Amboss wesentlich geschädigt ist beziehungsweise gar nicht mehr vorhanden ist und somit ein derartiger Wandler nicht mehr mechanisch an ein schwingfähiges und mit dem Innenohr in Verbindung stehendes Ossikel angekoppelt werden kann.

Ein gewisser Nachteil der Wandlervarianten nach b) ist der Umstand, dass die Wandlergehäuse mit implantierbaren Positionier- und Fixationssystemen (Mikromanipulatoren) an der Schädelkalotte befestigt werden müssen (vorteilhafte Ausführung US-A 5 788 711). Ein weiterer Nachteil der Varianten nach b) besteht darin, dass, vorzugsweise mittels geeigneter Laser, Vertiefungen in die Zielossikel eingebracht werden müssen, um das Koppelelement applizieren zu können. Dies ist einerseits technisch aufwendig und teuer und bringt andererseits Risiken für den Patienten mit sich. Sowohl bei dem teilimplantierbaren System nach FREDRICKSON ("Ongoing investigations into an implantable elektromagnetic hearing aid for moderate to severe sensorineural hearing loss"; Otolaryngologic Clinics Of North America, Vol. 28/1 (1995), pp. 107-121) wie auch bei dem vollimplantierbaren Hörsystem nach LEYSIEFFER und ZENNER (HNO 1998, Vol. 46, 853-863 und 844-852) wird bei der Ankopplung des schwingenden Wandlerteils an den Ambosskörper zur dauerhaften und mechanisch sicheren Schwingungsübertragung davon ausgegangen, dass die Spitze der Koppelstange, die in die laserinduzierte Vertiefung des Mittelohrossikels eingebracht wird, langfristig eine Osseointegration erfährt, das heißt, die Koppelstange verwächst fest mit dem Ossikel und gewährleistet so eine sichere Übertragung dynamischer Druck- und Zugkräfte. Dieser Langzeiteffekt ist zur Zeit jedoch noch nicht wissenschaftlich nachgewiesen beziehungsweise gesichert. Weiterhin besteht bei dieser Ankopplungsart bei einem technischen Wandlerdefekt der Nachteil, dass eine Entkopplung vom Ossikel zur Entfernung des Wandlers nur mit mechanisch basierten operativen Methoden erfolgen kann, was eine erhebliche Gefährdung des Mittelohres und insbesondere des Innenohres bedeuten kann. Daher wurden weitere Koppelelemente mit zum Teil neuen operativen Zugangswegen entwickelt, die diese genannten Nachteile minimieren oder nicht mehr aufweisen und auf die weiter unten näher eingegangen ist.

Der wesentliche Vorteil dieser Wandlerausführungsformen nach b) besteht jedoch darin, dass das Mittelohr weitgehend frei bleibt und der Zugang zum Mittel- und Innenohr so erfolgen kann, dass bei entsprechender Auslegung des Mikromanipulators prinzipiell jeder Punkt des Mittelohres beziehungsweise Innenohres als Zielstimulationsort erreichbar ist. Daraus resultiert als weiterer Vorteil, dass grundlegend alle Kombinationen von Mittel- und Innenohrschäden versorgbar sind und auch eine reine Innenohrstimulation über einen direkten Zugang zum Beispiel über eine artifizielle Fensterung und Verwendung geeigneter Koppelelemente möglich ist. Dazu ist auch eine lösbare Verbindungsmechanik zwischen Wandler und Koppelelement angegeben worden (DE-A-199 48 336).

Aus WO 00/48426 ist ein Hörsystem bekannt, bei dem eine aus einem aktorischen elektromechanischen Wandler und einem Positionier- und Fixationssystem (vorliegend "Mikromanipulator" genannt) bestehende Einheit mit einer am Schädel fest montierten Halterung lösbar verbunden ist, so dass im Bedarfsfall der Mikromanipulator zusammen mit dem Wandler ausgetauscht werden kann, ohne dass die Halterung abgebaut werden muss. Das Entfernen der Halterung kann nämlich einen relativ invasiven Eingriff erfordern, da der operative Zugang zu den Befestigungsschrauben des Mikromanipulators freigelegt werden muss. Weiterhin kann es vorkommen, dass die entsprechenden Schraubenlöcher in der Schädelkalotte nicht mehr wiederverwendet werden können, da die Schrauben in der Regel selbstschneidend ausgelegt sind und das entsprechende Loch im Knochen beim Aufdrehen erweitert wird und somit nicht mehr dem Anspruch des absolut festen Sitzes der neuen Schraube genügen kann. Es kann auch der Fall eintreten, dass der "alte" Zugangsweg zum Zielort des Wandler-Koppelelementes nicht mehr oder nur stark eingeschränkt verwendbar ist. Die bekannte Lösung lässt jedoch noch immer zu wünschen übrig. So muss dort nach einem Austausch der Mikromanipulator/Wandler-Einheit diese Einheit in umständlicher Weise wieder neu eingestellt werden, um den Wandler mit Bezug auf den Zielort der Stimulation exakt auszurichten. Dies macht vor allem dann, wenn ein Wandler postoperativ defekt geworden und daher ein operativer Austausch des Wandlers erforderlich wird, die Reversions-Operation deutlich risikoreicher und verlängert insbesondere eine solche Operation. Es kann dazu kommen, dass der sonst eventuell in lokaler Anästhesie durchführbare Eingriff in totaler Anästhesie erfolgen muss, weil die Operationstiefe und -dauer dies erfordern. Die feste Verbindung von Wandler und Mikromanipulator hat ferner den technischen Nachteil, dass kaum eine konstruktive Lösung für beide Komponenten gefunden werden kann, die eine Seitigkeit des Systems (Links-Rechts-Unterscheidung) vermeidet. Daraus resultiert auch der wirtschaftliche Nachteil, dass bei jeder Neuimplantation zwei komplette Wandler-Mikromanipulator-Systeme geliefert werden müssen, da möglicherweise erst kurz vor der Operation die betreffende Seite zusammen mit dem Patienten beschlossen wird. Ein weiterer wirtschaftlicher Nachteil besteht bei einer Reversionsoperation wegen einem Wandlerdefekt wie oben angegeben darin, dass ein völlig funktionstüchtiger Mikromanipulator explantiert und weggeworfen werden muss, da dieser aufgrund der bestehenden Gesetzeslage nicht wieder verwendet werden darf. Ein entscheidender weiterer Nachteil besteht darin, dass bei zu den erwartenden Weiterentwicklungen der elektromechanischen Wandler implantierbarer Hörsysteme diese verbesserten Produkte einem bereits implantierten Patienten auch nur mit einem kompletten Systemwechsel Wandler/Mikromanipulator angeboten werden können.

Der Erfindung liegt die Aufgabe zugrunde, ein mindestens teilweise implantierbares Hörsystem zu schaffen, das die bei einem Defekt des ausgangsseitigen elektromechanischen Wandlers notwendig werdenden Maßnahmen vereinfacht und das gegebenenfalls auch relativ einfach auf verbesserte Wandler umgerüstet werden kann.

Ausgehend von einem mindestens teilweise implantierbaren Hörsystem mit mindestens einem ausgangsseitigen elektromechanischen Wandler und einem an der Schädelkalotte mittels einer Befestigungsanordnung fest montierbaren Mikromanipulator zum Positionieren des Wandlers und zum Fixieren des Wandlers in einer mittels des Mikromanipulators eingestellten Position wird diese Aufgabe erfindungsgemäß dadurch gelöst, dass zwischen dem Wandler und dem Mikromanipulator eine lösbare Kupplung angeordnet ist, die in einem montierten Zustand den Wandler mit Bezug auf den Mikromanipulator festlegt und die bei Lösen ein Abnehmen des Wandlers von dem Mikromanipulator erlaubt.

Bei Auftreten eines postoperativen Wandlerdefekts lässt sich unter Nutzung der gemäß der Erfindung vorgesehenen lösbaren Kupplung der Wandler allein, das heißt ohne den Mikromanipulator, austauschen. Entsprechendes gilt auch, wenn ein Wechsel des Wandlers aus anderen Gründen wünschenswert wird, beispielsweise wenn Weiterentwicklungen der Wandlertechnologie verbesserte Wandler verfügbar machen. Die mittels des Mikromanipulators eingestellte Wandlerposition bleibt auch bei einem Wandleraustausch erhalten, so dass es keiner erneuten Einstellung des Mikromanipulators bedarf. Weil der Wandler allein ausgewechselt werden kann, entfallen auch die vorstehend geschilderten Probleme hinsichtlich der Rechts/Links-Seitigkeit.

In weiterer Ausgestaltung der Erfindung weist die lösbare Kupplung ein wandlerseitiges Kupplungselement und ein mikromanipulatorseitiges Kupplungselement auf, die wahlweise in und außer mechanischen Eingriff miteinander bringbar sind. Dabei kann das wandlerseitige Kupplungselement so ausgebildet sein, dass es mit dem Wandler bereits bei der Produktion des Wandlers fest verbunden, zum Beispiel verklebt, wird, oder dass für eine solche feste Verbindung, beispielsweise in Form einer unlösbaren Verrastung, erst im Zuge der Implantation des Wandlers gesorgt wird.

Das mikromanipulatorseitige Kupplungselement kann zweckmäßig eine Aufnahme für das wandlerseitige Kupplungselement bilden.

Im Rahmen der Erfindung kann mindestens eines der beiden Kupplungselemente mindestens partiell aus elastischem Werkstoff, insbesondere einem weichen Polymer, vorzugsweise Silicon, Polytetrafluorethylen oder Polyurethan, gefertigt sein. Dadurch lässt sich eine Körperschallrückleitung vom Wandler auf den Schädel verhindern oder mindestens verringern. Je nach der konstruktiven Ausgestaltung der Kupplung können aber auch beide Kupplungselemente aus nichtelastischem Werkstoff, insbesondere einem harten Polymer, wie Polycarbonat, einem biokompatiblen Metall oder einem keramischen Werkstoff bestehen,

Die lösbare Kupplung kann als Rastkupplung ausgebildet sein, bei der beispielsweise das mikromanipulatorseitige Kupplungselement eine starre ringförmige Aufnahme ist, in welche ein mindestens partiell elastisches wandlerseitiges Kupplungselement in Axialrichtung einrastbar ist. Bei einer abgewandelten Ausführungsform einer Rastkupplung ist das mikromanipulatorseitige Kupplungselement als aufspreizbare Gabel ausgebildet, in welche das wandlerseitiges Kupplungselement in Radialrichtung einrastbar ist.

Das mikromanipulatorseitige Kupplungselement kann ferner eine aufspreizbare Aufnahme bilden, in die das wandlerseitige Kupplungselement in Axialrichtung einführbar ist, und die in einer das wandlerseitige Kupplungselement festhaltenden Stellung verrastbar ist.

Gemäß einer weiteren Ausgestaltung der Erfindung kann das mikromanipulatorseitige Kupplungselement auch als aufspreizbare Zange ausgebildet sein, in welche das wandlerseitige Kupplungselement in Axialrichtung einführbar ist, wobei vorzugsweise die aufspreizbare Zange in einer das wandlerseitige Kupplungselement festhaltenden Schließstellung verriegelbar ist, zum Beispiel mittels einer entlang einem Teil der Zange verschiebbaren Schiebehülse.

Entsprechend einer abgewandelten Ausführungsform kann die lösbare Kupplung als Steckkupplung mit zwei Kupplungselementen ausgebildet sein, von denen das eine in das andere einsteckbar ist und die im montierten Zustand über eine Presspassung miteinander in Eingriff gehalten sind. Dabei kann das eine Kupplungselement zweckmäßig einen schwalbenschwanzförmigen Teil aufweisen, der in eine komplementäre Aufnahmenut des anderen Kupplungselements einschiebbar ist.

Der Mikromanipulator, der Wandler und die Kupplungselemente sind vorzugsweise so konstruiert, dass eine Links/Rechts-Seitigkeit vermieden wird, das heißt die gleiche Kombination aus Mikromanipulator und Wandler sowohl für das rechte Ohr als auch für das linke Ohr verwendet werden kann. Dazu kann mindestens eines der beiden Kupplungselemente rotationssymmetrisch ausgebildet sein und/oder das mikromanipulatorseitige Kupplungselement kann spiegelsymmetrisch mit Bezug auf die Wandlerachse ausgebildet sein.

Im Rahmen der vorliegenden Erfindung können beliebige Wandlerprinzipien zum Einsatz kommen. Insbesondere kann der ausgangsseitige elektromechanische Wandler als elektromagnetischer, elektrodynamischer, magnetostriktiver, dielektrischer und/oder piezoelektrischer Wandler ausgebildet sein.

Die erfindungsgemäße Lösung kann unilateral (monaural) ebenso wie bilateral (binaural) eingesetzt werden, und die konstruktive Ausgestaltung kann so getroffen sein, dass sich die Kupplung wahlweise von Hand oder unter Verwendung eines zweckentsprechenden Werkzeugs schließen und öffnen lässt.

Bevorzugte Ausführungsbeispiele der erfindungsgemäßen Anordnung sind nachstehend unter Bezugnahme auf die beiliegenden Zeichnungen näher beschrieben. Es zeigen:
- FIG. 1: einen schematischen Schnitt durch ein menschliches Felsenbein mit in einer Mastoidhöhle befindlichem Wandler,
- FIG. 2: einen implantierbaren Mikromanipulator und einen zugehörigen Wandler,
- FIG. 3: die Anordnung des Mikromanipulators mit Wandler in der Mastoidhöhle,
- FIG. 4: eine Ausführungsform einer lösbaren Rastkupplung zwischen Mikromanipulator und Wandler,
- FIG. 5: eine weitere Ausführungsform einer lösbaren Rastkupplung zwischen Mikromanipulator und Wandler,
- FIG. 6: eine Ausführungsform einer lösbaren Steckkupplung zwischen Mikromanipulator und Wandler,
- FIG. 7: eine weitere Ausführungsform der lösbaren Steckkupplung zwischen Mikromanipulator und Wandler,
- FIG. 8: eine mögliche Ausführungsform der lösbaren Kupplung zwischen Mikromanipulator und Wandler mit einem eine aufspreizbare Aufnahme bildenden mikromanipulatorseitigen Kupplungselement,
- FIG. 9: eine mögliche Ausführungsform der lösbaren Kupplung zwischen Mikromanipulator und Wandler mit einem mikromanipulatorseitigen Kupplungselement nach Art einer in einer Schließstellung verriegelbaren aufspreizbaren Zange,
- FIG. 10: ein vollimplantierbares Hörsystem und
- FIG. 11: ein teilimplantierbares Hörsystem.

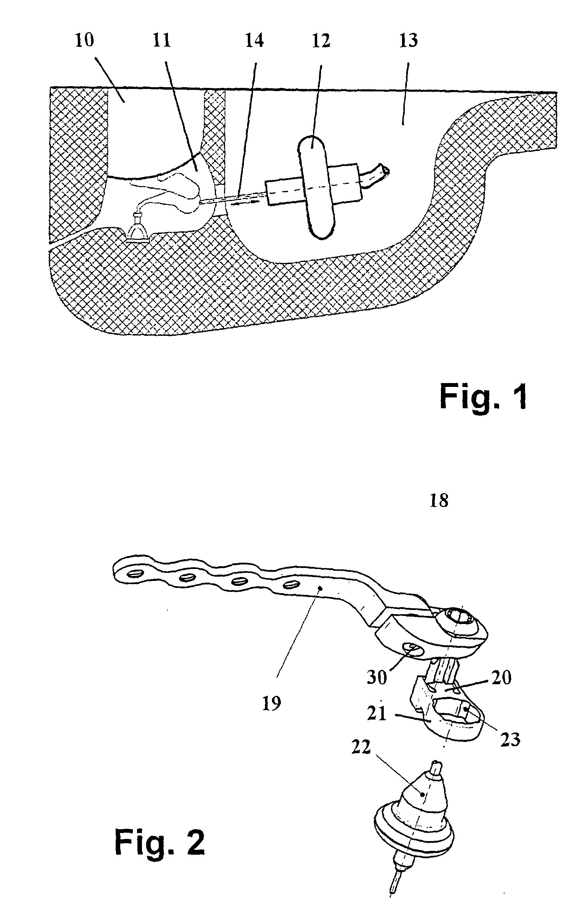

FIG. 1 zeigt schematisch einen Schnitt durch ein menschliches Felsenbein mit dem äußeren Gehörgang 10, dem Mittelohr 11 mit den Ossikeln Hammer, Amboss und Steigbügel (Innenohr nicht dargestellt) und einem elektromechanischen Wandler 12 in einer artifiziell geschaffenen Mastoidhöhle 13. Der Wandler 12 wird in an sich bekannter Weise zu mechanischen Schwingungen angeregt, und er überträgt in diesem Beispiel diese ausgangsseitigen mechanischen Schwingungen über eine sich durch den natürlichen Zugang aditus ad antrum erstreckende Koppelstange 14 auf den Amboss. Das implantierbare Positionier- und Fixationssystem (Mikromanipulator) ist hier nicht dargestellt.

FIG. 2 zeigt beispielhaft einen implantierbaren Mikromanipulator 18, wie er im einzelnen in DE-A-199 15 684 beschrieben ist. Der Mikromanipulator 18 weist eine zur Verschraubung an der Schädelkalotte bestimmte Osteosyntheseplatte 19 und einen Wandleraufnahmeschlitten 20 mit einem mikromanipulatorseitigen Kupplungselement 21 auf. Das Kupplungselement 21 bildet eine Aufnahme 23 für den Wandler 12. Es hat bei diesem Ausführungsbeispiel die Form eines geschlossenen Ringes. Ein wandlerseitiges Kupplungselement 22 ist mit dem Wandler 12 verbunden und bildet zusammen mit dem mikromanipulatorseitigen Kupplungselement 21 eine lösbare Kupplung. Der mit dem Kupplungselement 22 verbundene Wandler 12 kann in die Aufnahme 23 eingebracht und dort lösbar fixiert werden.

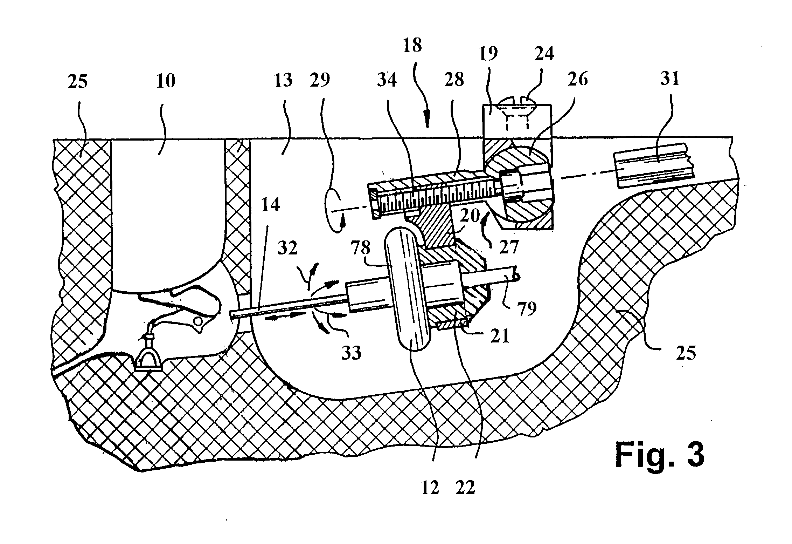

FIG. 3 zeigt Details des Mikromanipulators 18 und dessen Anordnung mit einem daran lösbar fixierten elektromechanischen Wandler 12 im implantierten Zustand in der Mastoidhöhle 13. Mittels Knochenschrauben 24 ist die Osteosyntheseplatte 19 auf der Schädelkalotte 25 festgeschraubt. Die Osteosyntheseplatte 19 ist über ein mit Gelenkkugel 26 versehenes Kugelgelenk 27 mit einer geraden Führungsschiene 28 drehbar (Pfeil 29) und schwenkbar verbunden. Das Kugelgelenk 27 kann durch Anziehen einer Klemmschraube 30 (FIG. 2) festgeklemmt werden. Durch Lösen der Klemmschraube 30 ist die Gelenkkugel 26 in ihrem Kugelsitz mittels eines Hilfswerkzeuges 31 in allen drei rotatorischen Freiheitsgraden 29, 32 und 33 frei schwenkbar. An dem aus Gelenkkugel 26 und Führungsschiene 28 bestehenden Bauteil ist eine Gewindespindel 34 drehbar, aber in Axialrichtung unverschiebbar gelagert. Mit einem Außengewinde der Gewindespindel 34 steht ein Innengewinde einer Spindelmutter in Eingriff, die Teil des Wandleraufnahmeschlittens 20 ist. Durch Drehen der Gewindespindel 34 mittels eines nicht dargestellten Hilfswerkzeuges kann der Schlitten 20 zusammen mit dem in der Aufnahme 23 sitzenden Wandler 12 axial entlang der Führungsschiene 28 verschoben werden. Bei der veranschaulichten Ausführungsform haben der Wandler 12 und die Koppelstange 14 eine Längsachse, die mindestens näherungsweise parallel zu der Längsachse der Gewindespindel 34 und der Führungsschiene 28 verläuft, gegenüber der Längsachse der Gewindespindel aber in Querrichtung versetzt ist.

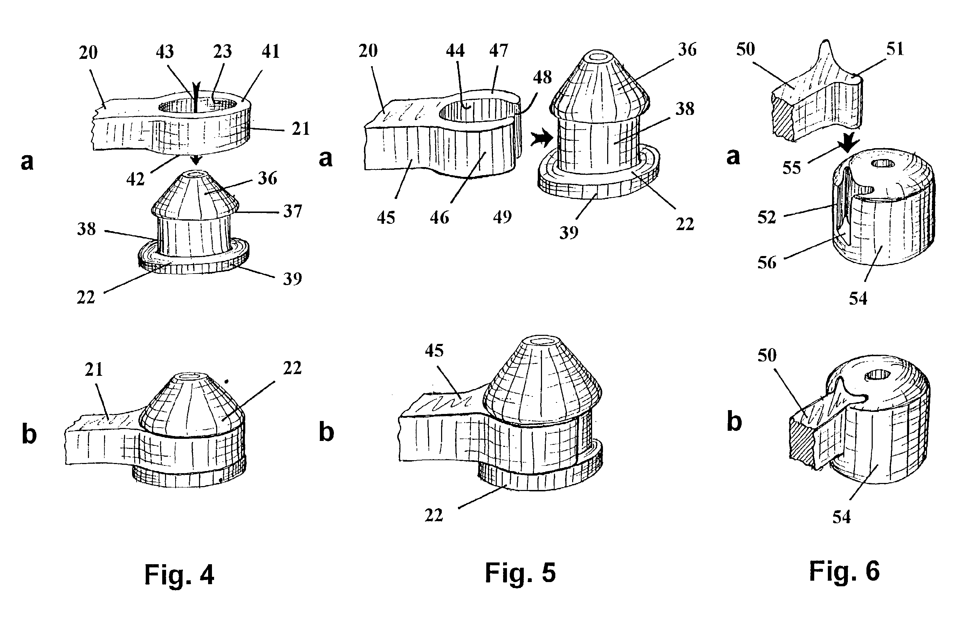

Die Figuren 4a und 4b zeigen eine mögliche Ausführungsform einer als Rastkupplung ausgestalteten lösbaren Kupplung zwischen Mikromanipulator 18 und Wandler 12. Dabei ist das wandlerseitige Kupplungselement 22 als elastische Tülle ausgeführt, und es weist in Axialrichtung aufeinanderfolgend einen kegelstumpfförmigen Kopf 36 mit einer Kegelstumpf-Grundfläche 37, einen kreiszylindrischen Hals 38 und einen Kragen 39 auf. Die Kegelstumpf-Grundfläche 37 des Kopfes 36 hat einen Durchmesser, der etwas größer als der Durchmesser des Halses 38 ist. Der Kragen 39 springt an der von dem Kopf 36 abliegenden Seite des Halses 38 radial nach außen vor. Die von dem mikromanipulatorseitigen Kupplungselement 21 gebildete Aufnahme 23 ist kreiszylindrisch, und sie hat einen Innendurchmesser, der etwa gleich dem Außendurchmesser des Halses 38, aber kleiner als der Durchmesser der Kegelstumpf-Grundfläche 37 und des Kragens 39 ist. Die Axialabmessung des Halses 38 des wandlerseitigen Kupplungselements 22 ist mindestens näherungsweise gleich der Axialabmessung der Wandleraufnahme 23 des Mikromanipulators 18.

Das elastische wandlerseitige Kupplungselement 22 kann - unter leichter Verformung des Kopfes 36 - in Axialrichtung in die Aufnahme 23 des mikromanipulatorseitigen Kupplungselements 21 eingeschoben werden. Sobald dabei die Kegelstumpf-Grundfläche 37 des Kupplungselementkopfes 36 aus der Aufnahme 23 (in den Figuren 4a und 4b nach oben) wieder austritt, ist das Kupplungselement 22 in die Aufnahme 23 mechanisch sicher eingerastet, wobei die Aufnahme 23 den Hals 38 umschließt und die einander zugewendeten Stirnflächen von Kopf 36 und Kragen 39 sich im Bereich der Aufnahme 23 gegen die Seitenflächen 41 beziehungsweise 42 des Schlittens 20 anlegen (FIG. 4b). Zum Lösen des Wandlers 12 von dem Mikromanipulator 18 kann der Kopf 36 des Kupplungselements 22 mit einem entsprechenden Werkzeug oder von Hand leicht zusammengedrückt und das Kupplungselement 22 aus dem Schlitten 20 gezogen werden, wie dies in FIG. 4a mit dem Pfeil 43 angedeutet ist.

Abgewandelte Ausführungsbeispiele sind in den Figuren 5 bis 9 dargestellt, wobei wiederum jeweils das Teilbild a den gelösten Zustand und Teilbild b den montierten Zustand zeigt.

FIG. 5 zeigt eine weitere mögliche Ausführungsform einer lösbaren Rastkupplung zwischen dem Mikromanipulator 18 und dem Wandler 12 gemäß FIG. 1. Dabei weist der Schlitten 20 ein mikromanipulatorseitiges Kupplungselement 45 auf, das gabelförmig gestaltet ist und eine einseitig radial offene Aufnahme 44 für das mit dem Wandler 12 verbundene Kupplungselement 22 bildet. Gabelarme 46,47 von im wesentlichen gleicher Länge begrenzen mit ihren freien Enden eine Gabelöffnung 48, deren Weite um ein vorbestimmtes Maß kleiner als der Außendurchmesser des Halses 38 des wandlerseitigen Kupplungselements 22 ist. Die Gabelarme 46, 47 können starr ausgebildet sein, wenn das Kupplungselement 22 mindestens in seinem Halsbereich elastisch ist. Das Kupplungselement 22 kann aber auch starr sein, wenn das Kupplungselement 45 leicht federnd konstruiert ist. Ferner können sowohl die Gabel 45 als auch das wandlerseitige Kupplungselement 22 elastisch nachgiebig sein. Im einen wie im anderen Fall kann das mit dem Wandler 12 in nicht näher dargestellter Weise verbundene Kupplungselement 22 durch bezüglich der Kupplungselementachse radiale Bewegung in die Aufnahme 44 eingerastet beziehungsweise von dieser gelöst (Pfeil 49) werden.

FIG. 6 zeigt eine mögliche Ausführungsform einer lösbaren Steckkupplung zwischen dem Mikromanipulator 18 und dem Wandler 12 gemäß FIG. 1. Der Schlitten 20 weist dabei abweichend von der Darstellung in den Figuren 2 und 3 ein mikromanipulatorseitiges Kupplungselement 50 auf, das sich an seinem freien Ende 51 schwalbenschwanzförmig erweitert und das in eine komplementär gestaltete, einseitig (in FIG. 6 oben) offene Aufhahmenut 52 eines mit dem Wandler 12 (nicht dargestellt) verbundenen Kupplungselements 54 einschiebbar ist (Pfeil 55). Eine dem offenen Ende der Aufhahmenut 52 gegenüberliegende Endwand 56 der Aufhahmenut 52 bildet dabei einen Anschlag für das Kupplungselement 50. In diesem Beispiel können sowohl das wandlerseitige Kupplungselement 54 als auch das mikromanipulatorseitige Kupplungselement 50 mechanisch starr ausgeführt sein. Die Verbindung zwischen Wandler und Mikromanipulator erfolgt dann durch einen kombinierten Form- und Reibschluss. Stattdessen kann mindestens eines der Elemente 50, 54 mindestens im jeweiligen Koppelbereich elastisch ausgeführt sein.

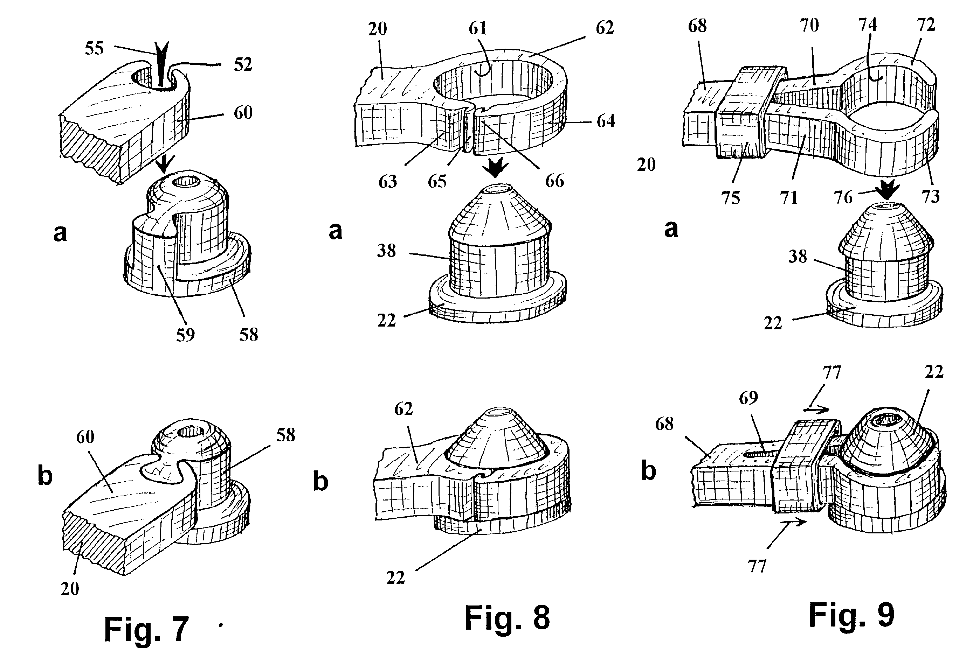

Bei der Ausführungsform gemäß FIG. 7 ist ein wandlerseitiges Kupplungselement 58 mit einem schwalbenschwanzförmigen Teil 59 versehen, und die komplementäre Aufhahmenut 52 ist in einem mikromanipulatorseitigen Kupplungselement 60 des Wandleraufnahmeschlittens 20 ausgebildet. Abgesehen von dieser Vertauschung von schwalbenschwanzförmigem Teil und Schwalbenschwanz-Aufnahmenut entspricht die Lösung gemäß FIG. 7 derjenigen nach FIG. 6.

Im Falle des Ausführungsbeispiels der FIG. 8 weist die lösbare Kupplung zwischen Mikromanipulator 18 und Wandler 12 gemäß FIG. 1 ein mikromanipulatorseitiges Kupplungselement 62 auf, das eine aufspreizbare, in einer Schließstellung verrastbare, ringförmige Aufnahme 61 bildet. Das mikromanipulatorseitige Kupplungselement 62 ist mit einem kürzeren Schenkel 63 und einem längeren Schenkel 64 versehen. Die Schenkel 63, 64 sind an ihrem freien Ende 65 beziehungsweise 66 jeweils komplementär hakenförmig gestaltet. Die Haken 65, 66 können zum Aufspreizen der Aufnahme 61 und zum Lösen des Wandlers 12 von dem Mikromanipulator 18 außer Eingriff miteinander gebracht werden (FIG. 8a). In diesem Zustand befinden sich die Haken auch zunächst beim Anmontieren des Wandlers 12 an den Mikromanipulator 18. Nachdem dann der Hals 38 des wandlerseitigen Kupplungselements 22 in die Aufnahme 61 eingebracht ist, wird der längere Schenkel 64 derart zusammengedrückt, dass die Haken 65, 66 miteinander verrastet werden können (FIG. 8b). In diesem Beispiel kann das wandlerseitige Kupplungselement 22 mechanisch starr oder elastisch ausgeführt sein. Mindestens der längere Schenkel 64 des mikromanipulatorseitigen Kupplungselements 62 ist federnd ausgelegt.

FIG. 9 zeigt eine weitere Ausführungsform der lösbaren Kupplung zwischen Mikromanipulator 18 und Wandler 12. Dabei ist ein mikromanipulatorseitiges Kupplungselement 68 des Schlittens 20 als aufspreizbare Zange ausgebildet, die dadurch erhalten wird, dass ein Längsschlitz 69 den von der Spindel 34 abliegenden Teil des Schlittens 20 in zwei federnde Arme 70, 71 teilt. Freie Enden 72, 73 der Arme 70, 71 bilden eine bei geschlossener Zange ringförmige Aufnahme 74 für das wandlerseitige Kupplungselement 22. Der Schlitz 69 erstreckt sich von der Aufnahme 74 in Richtung auf die Führungsschiene 28 des Mikromanipulators 18. Die Arme 70, 71 sind in eine aufgespreizte Stellung (FIG. 9a) federnd vorgespannt, in welcher ihre freien Enden 72, 73 eine Lage einnehmen, die ein Einsetzen des wandlerseitigen Kupplungselements 22 in die Aufnahme 74 und ein Lösen des Kupplungselements 22 von der Aufnahme 74 (Pfeil 76 in FIG. 9a) gestattet. Eine Schiebehülse 75 umgreift das Kupplungselement 68. Die Schiebehülse 75 ist entlang dem Kupplungselement 68 zwischen einer die Arme 70, 71 zum Aufspreizen freigebenden Stellung (FIG. 9a) und einer Stellung näher den freien Enden 72, 73 verschiebbar (Pfeile 77 in FIG. 9b). In der letztgenannten Stellung presst die Schiebehülse 75 die Arme 70, 71 entgegen der Federvorspannkraft aufeinander zu, bis die Federarmenden 72, 73 den Hals 38 des wandlerseitigen Kupplungselements 22 umschließen und das wandlerseitige Kupplungselement samt dem damit verbundenen Wandler 12 (nicht dargestellt) sicher festhalten (FIG. 9b). In diesem Ausführungsbeispiel kann das wandlerseitige Kupplungselement 22 mechanisch starr oder elastisch ausgeführt sein.

Bei allen vorstehend erläuterten Ausführungsbeispielen können der Wandler 12 und das wandlerseitige Kupplungselement 22 beziehungsweise 54 beziehungsweise 58 fest miteinander verbunden, zum Beispiel während der Produktion miteinander mechanisch verrastet oder fest verklebt, sein. Auch eine einstückige Ausbildung von Wandler Wandlerkupplungselement ist möglich. Es kann aber auch vorgesehen sein, dass das wandlerseitige Kupplungselement erst im Zuge der Implantation des Wandlers mit diesem fest verbunden, beispielsweise verrastet, wird. Im Austauschfall wird dann zum Beispiel dieses wandlerseitige Kupplungselement als austauschbares Einzelteil mit dem neuen Wandler in einer Sterilverpackung mitgeliefert.

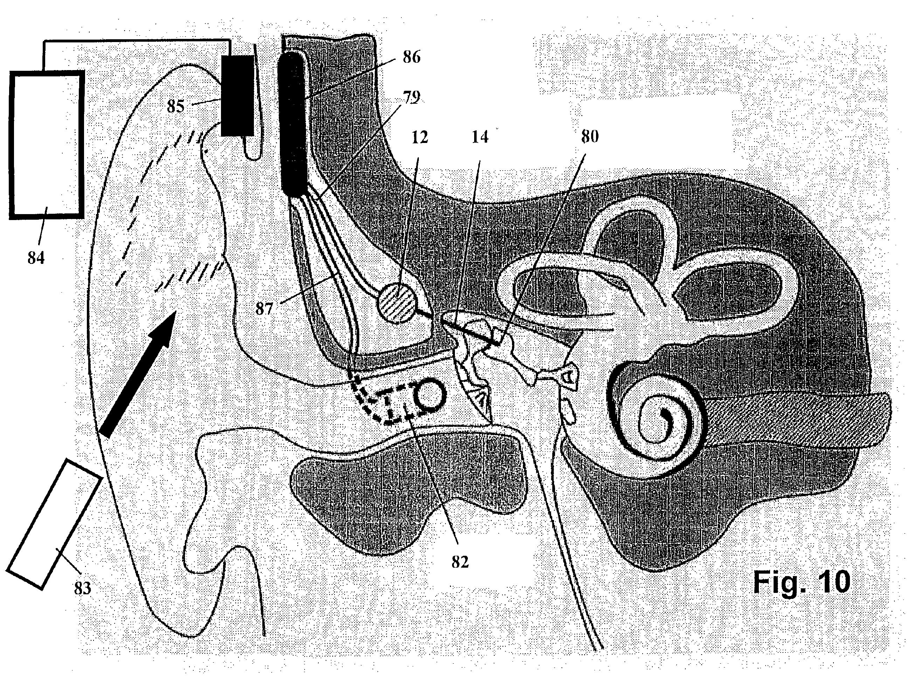

FIG. 10 zeigt den Aufbau eines vollständig implantierbaren Hörsystems, das als aktorische Stimulationsanordnung einen ausgangsseitigen elektromechanischen Wandler 12 aufweist, der über ein wandlerseitiges Kupplungselement 22 beziehungsweise 54 beziehungsweise 58 mit dem in FIG. 10 nicht dargestellten Mikromanipulator 18 (siehe insbesondere FIG. 3) lösbar gekuppelt ist. Der Wandler 12 kann allgemein als beliebiger elektromagnetischer, elektrodynamischer, piezoelektrischer, magnetostriktiver oder dielektrischer (kapazitiver) Wandler ausgebildet sein. Eine bevorzugte Ausführungsform eines piezoelektrischen Wandlers ist aus US-A-5 277 694 bekannt. Ein solcher Wandler weist ein biokompatibles, zylindrisches Gehäuse 78 (FIG. 3) aus elektrisch leitendem Material, beispielsweise Titan, auf, das mit Inertgas gefüllt ist. In dem Gehäuse 78 ist eine schwingungsfähige, elektrisch leitende Membran angeordnet. Die Membran ist vorzugsweise kreisrund, und sie ist an ihrem Außenrand mit dem Gehäuse 78 fest verbunden. An der einen Seite der Membran sitzt eine dünne Scheibe aus piezoelektrischem Material, zum Beispiel Blei-Zirkonat-Titanat (PZT). Die der Membran zugewendete Seite der Piezoscheibe steht mit der Membran in elektrisch leitender Verbindung. Das Anlegen einer elektrischen Spannung an die Piezoscheibe über eine Wandlerzuleitung 79 bewirkt ein Durchbiegen des Hetero-Verbundes aus Membran und Piezoscheibe und führt somit zu einer Auslenkung der Membran und der an der Membran befestigten Koppelstange 14. Ein Wandler 12 dieser Art hat typischerweise eine relativ hohe mechanische Ausgangsimpedanz, insbesondere eine mechanische Ausgangsimpedanz, die höher ist als die mechanische Lastimpedanz der im implantierten Zustand an den Wandler angekoppelten biologischen Mittel- und/oder Innenohrstruktur. Der Wandler 12 kann unter anderem auch in der in DE-C-198 40 211 erläuterten Weise dahingehend modifiziert sein, dass an der von der Membran abgewendeten Seite der Piezoscheibe ein Permanentmagnet angebracht ist, der nach Art eines elektromagnetischen Wandlers mit einer Elektromagnetspule zusammenwirkt. Ein solcher kombinierter piezoelektrischer/elektromagnetischer Wandler ist besonders im Hinblick auf ein breites Frequenzband und auf die Erzielung relativ großer Schwingungsamplituden mit verhältnismäßig kleiner zugeführter Energie von Vorteil. Bei dem Wandler 12 kann es sich ferner um eine elektromagnetische Wandleranordnung handeln, wie sie in EP-A-0 984 663 beschrieben ist.

Zum Ankoppeln des Wandlers 12 an das Mittel- oder Innenohr (zum Beispiel Ankoppelstelle 80 an den Amboss) eignen sich besonders Koppelanordnungen gemäß US-A-5 941 814, bei denen ein Koppelelement außer einem Ankoppelteil für den betreffenden Ankoppelort eine Crimphülse aufweist, die zunächst lose auf einen mit rauer Oberfläche versehenen stabförmigen Teil einer Koppelstange aufgeschoben ist, die in der zuvor erläuterten Weise mit dem Wandler verbunden ist. Beim Implantieren kann die Crimphülse gegenüber der Koppelstange einfach verschoben und gedreht werden, um das Ankoppelteil des Koppelelementes mit dem beabsichtigten Ankoppelort exakt auszurichten. Dann wird die Crimphülse fixiert, indem sie mittels eines Crimpwerkzeuges plastisch kaltverformt wird. Alternativ kann das Koppelelement mit Bezug auf die Koppelstange auch mittels einer zuziehbaren Bandschlaufe festgelegt werden.

Weitere vorliegend bevorzugt verwendbare Koppelanordnungen sind im einzelnen in DE-A-199 23 403, DE-A-199 35 029, DE-C-199 31 788, DE-A-199 48 336 und DE-A-199 48 375 beschrieben. So kann gemäß DE-A-199 23 403 ein Koppelelement an seinem Ankoppelende eine Kontaktfläche aufweisen, die eine an die Oberflächenform der Ankoppelstelle anpassbare oder angepasste Oberflächenform sowie eine solche Oberflächenbeschaffenheit und Oberflächengröße aufweist, dass es durch Anlegen des Ankoppelendes an die Ankoppelstelle zu einer dynamischen Zug-Druck-Kraftkopplung von Koppelelement und Ossikelkette durch Oberflächenadhäsion kommt, die für eine sichere gegenseitige Verbindung von Koppelelement und Ossikelkette ausreicht. Das Koppelelement kann mit einem im implantierten Zustand an der Ankoppelstelle anliegenden Dämpfungsglied mit entropieelastischen Eigenschaften versehen sein, um eine optimale Schwingungsform der Steigbügelfußplatte oder einer das runde Fenster oder ein artifizielles Fenster in der Cochlea, im Vestibulum oder im Labyrinth abschließenden Membran zu erreichen und das Risiko einer Beschädigung der natürlichen Strukturen im Bereich der Ankoppelstelle während und nach der Implantation besonders gering zu halten (DE-A-199 35 029).

Das Koppelelement kann entsprechend DE-C-199 31 788 mit einer Stellvorrichtung zum wahlweisen Verstellen des Koppelelements zwischen einer Offenstellung, in welcher das Koppelelement in und außer Eingriff mit der Ankoppelstelle bringbar ist, und einer Schließstellung versehen sein, in welcher das Koppelelement im implantierten Zustand mit der Ankoppelstelle in Kraft- und/oder Formschlussverbindung steht.

Zum mechanischen Ankoppeln des elektromechanischen Wandlers an eine vorgewählte Ankoppelstelle an der Ossikelkette eignet sich ferner eine Koppelanordnung (DE-A-199 48 336), die eine von dem Wandler in mechanische Schwingungen versetzbare Koppelstange sowie ein mit der vorgewählten Ankoppelstelle in Verbindung bringbares Koppelelement aufweist, wobei die Koppelstange und das Koppelelement über wenigstens eine Kupplung miteinander verbunden sind und zumindest ein im implantierten Zustand an der Ankoppelstelle anliegender Abschnitt des Koppelelements zur verlustarmen Schwingungseinleitung in die Ankoppelstelle ausgelegt ist, wobei eine erste Kupplungshälfte der Kupplung eine Außenkontur mit mindestens näherungsweise der Gestalt einer Kugelkalotte aufweist, die in einer zur Außenkontur wenigstens teilweise komplementären Innenkontur einer zweiten Kupplungshälfte aufnehmbar ist, und wobei die Kupplung gegen Reibkräfte reversibel verschwenk- und/oder drehbar, jedoch bei im implantierten Zustand auftretenden dynamischen Kräften im Wesentlichen starr ist. Entsprechend einer abgewandelten Ausführungsform einer solchen Koppelanordnung (DE-A-199 48 375) hat eine erste Kupplungshälfte der Kupplung eine Außenkontur mit mindestens näherungsweise zylindrischer, vorzugsweise kreiszylindrischer, Gestalt, die in einer zur Außenkontur wenigstens teilweise komplementären Innenkontur einer zweiten Kupplungshälfte aufnehmbar ist, wobei ein im implantierten Zustand an der Ankoppelstelle anliegender Abschnitt des Koppelelements zur verlustarmen Schwingungseinleitung in die Ankoppelstelle ausgelegt ist, wobei im implantierten Zustand eine Übertragung von dynamischen Kräften zwischen den beiden Kupplungshälften der Kupplung im Wesentlichen in Richtung der Längsachse der ersten Kupplungshälfte erfolgt, und wobei die Kupplung reversibel an- und abkuppelbar sowie reversibel linear und/oder rotatorisch mit Bezug auf eine Längsachse der ersten Kupplungshälfte verstellbar, jedoch bei im implantierten Zustand auftretenden dynamischen Kräften starr ist.

Zu dem in FIG. 10 dargestellten vollständig implantierbaren Hörsystem gehören ferner ein implantierbares Mikrofon (Schallsensor) 82, eine drahtlose Fernbedienung 83 zur Steuerung der Implantatfunktionen durch den Implantatträger sowie ein drahtloses, transkutanes Ladesystem mit einem Ladegerät 84 und einer Ladespule 85 zur Nachladung einer im Implantat befindlichen sekundären Batterie zur Energieversorgung des Hörsystems.

Das Mikrofon 10 kann vorteilhaft in der aus EP-A-0 831 673 bekannten Weise aufgebaut und mit einer Mikrofonkapsel, die in einem Gehäuse allseitig hermetisch dicht untergebracht ist, sowie mit einer elektrischen Durchführungsanordnung zum Durchführen mindestens eines elektrischen Anschlusses von dem Innenraum des Gehäuses zu dessen Außenseite versehen sein, wobei das Gehäuse mindestens zwei Schenkel aufweist, die in einem Winkel mit Bezug aufeinander ausgerichtet sind, wobei der eine Schenkel die Mikrofonkapsel aufnimmt und mit einer Schalleintrittsmembran versehen ist, wobei der andere Schenkel die elektrische Durchführungsanordnung enthält und gegenüber der Ebene der Schalleintrittsmembran zurückversetzt ist, und wobei die Geometrie des Mikrofongehäuses so gewählt ist, dass bei Implantation des Mikrofons in der Mastoidhöhle der die Schalleintrittsmembran enthaltende Schenkel vom Mastoid aus in eine artifizielle Bohrung in der hinteren, knöchernen Gehörgangswand hineinragt und die Schalleintrittsmembran die Haut der Gehörgangswand berührt. Zur Festlegung des implantierten Mikrofons 38 kann zweckmäßig ein Fixationselement der aus US-A-5 999 632 bekannten Art vorgesehen sein, das eine Manschette aufweist, die mit einem zylindrischen Gehäuseteil den die Schalleintrittsmembran enthaltenden Schenkel umschließt und mit gegen die der Gehörgangshaut zugewendete Seite der Gehörgangswand anlegbaren, vorspringenden, elastischen Flanschteile versehen ist. Dabei beinhaltet das Fixationselement vorzugsweise eine Halterung, welche die genannten Flanschteile vor der Implantation entgegen einer elastischen Rückstellkraft der Flanschteile in einer das Durchstecken durch die Bohrung der Gehörgangswand erlaubenden umgebogenen Stellung hält.

Die an den Ausgang des Ladegerätes 84 angeschlossene Ladespule 85 bildet vorzugsweise in der aus US-A-5 279 292 bekannten Art Teil eines Sende-Serienresonanzkreises, der mit einem nicht veranschaulichten Empfangs-Serienresonanzkreis induktiv gekoppelt werden kann. Der Empfangs-Serienresonanzkreis kann Teil eines implantierbaren Elektronikmoduls 86 sein und entsprechend US-A-5 279 292 eine Konstantstromquelle für die Batterie bilden. Dabei liegt der Empfangs-Serienresonanzkreis in einem Batterie-Ladestromkreis, der in Abhängigkeit von der jeweiligen Phase des in dem Ladestromkreis fließenden Ladestromes über den einen oder den anderen Zweig einer Vollweg-Gleichrichterbrücke geschlossen wird.

Das Elektronikmodul 86 ist bei der Anordnung nach FIG. 10 über eine Mikrofonleitung 87 an das Mikrofon 82 und über die Wandlerzuleitung 79 an den elektromechanischen Wandler 12 angeschlossen.

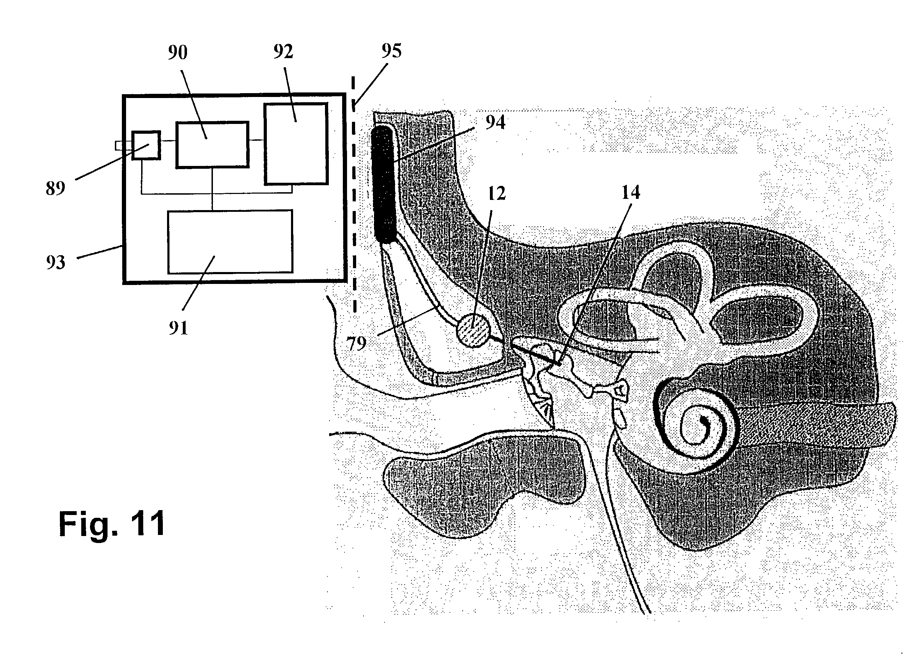

FIG. 11 zeigt den Aufbau eines teilimplantierbaren Hörsystems. Bei diesem teilimplantierbaren System sind ein Mikrofon 89, ein Elektronikmodul 90 für eine elektronische Signalverarbeitung, die Energieversorgung (Batterie) 91 sowie eine Modulator/Sender-Einheit 92 in einem extern am Körper, vorzugsweise am Kopfüber dem Implantat, zu tragenden externen Modul 93 enthalten. Das Implantat ist wie bei bekannten Teilimplantaten energetisch passiv. Sein Elektronikmodul 94 (ohne Batterie) empfängt Betriebsenergie und Steuersignale für den Wandler 12 über die Modulator/Sender-Einheit 92 im externen Teil 93 durch die geschlossene Haut 95 hindurch.

Sowohl das vollimplantierbare als auch das teilimplantierbare Hörsystem können monoaural (wie in den Figuren 10 und 11 dargestellt) oder binaural ausgelegt sein. Ein binaurales System zur Rehabilitation einer Hörstörung beider Ohren weist zwei Systemeinheiten auf, die jeweils einem der beiden Ohren zugeordnet sind. Dabei können die beiden Systemeinheiten einander im wesentlichen gleich sein. Es kann aber auch die eine Systemeinheit als Master-Einheit und die andere Systemeinheit als von der Master-Einheit gesteuerte Slave-Einheit ausgelegt sein. Die Signalverarbeitungsmodule der beiden Systemeinheiten können auf beliebige Weise, insbesondere über eine drahtgebundene implantierbare Leitungsverbindung oder über eine drahtlose Verbindung, vorzugsweise eine bidirektionale Hochfrequenzstrecke, eine körperschallgekoppelte Ultraschallstrecke oder eine die elektrische Leitfähigkeit des Gewebes des Implantatträgers ausnutzende Datenübertragungsstrecke, so miteinander kommunizieren, dass in beiden Systemeinheiten eine optimierte binaurale Signalverarbeitung erreicht wird.

Folgende Kombinationsmöglichkeiten sind vorsehbar:
- Beide Elektronikmodule können jeweils einen digitalen Signalprozessor gemäß vorstehender Beschreibung enthalten, wobei die Betriebssoftware beider Prozessoren wie beschrieben transkutan veränderbar ist. Dann sorgt die Verbindung beider Module im wesentlichen für den Datenaustausch zur optimierten binauralen Signalverarbeitung zum Beispiel der Sensorsignale.
- Nur ein Modul enthält den beschriebenen digitalen Signalprozessor, wobei dann die Modulverbindung neben der Sensordatenübertragung zur binauralen Schallanalyse und -verrechnung auch für die Ausgangsignalübermittlung zu dem kontralateralen Wandler sorgt, wobei in dem kontralateralen Modul der elektronische Wandlertreiber untergebracht sein kann. In diesem Fall ist die Betriebssoftware des gesamten binauralen Systems nur in einem Modul abgelegt und wird auch nur dort transkutan über eine nur einseitig vorhandene Telemetrieeinheit von extern verändert. In diesem Fall kann auch die energetische Versorgung des gesamten binauralen Systems in nur einem Elektronikmodul untergebracht sein, wobei die energetische Versorgung des kontralateralen Moduls drahtgebunden oder drahtlos geschieht.

## Patentansprüche

1. Mindestens teilweise implantierbares Hörsystem mit mindestens einem ausgangsseitigen elektromechanischen Wandler (12) und einem an der Schädelkalotte mittels einer Befestigungsanordnung (19) fest montierbaren Mikromanipulator (18) zum Positionieren des Wandlers und zum Fixieren des Wandlers in einer mittels des Mikromanipulators eingestellten Position, **gekennzeichnet durch** eine lösbare Kupplung (21, 45, 50, 60, 62, 68; 22, 54, 58) zwischen dem Wandler (12) und dem Mikromanipulator (18), die in einem montierten Zustand den Wandler mit Bezug auf den Mikromanipulator festlegt und die bei Lösen ein Abnehmen des Wandlers von dem Mikromanipulator erlaubt.

2. Hörsystem nach Anspruch 1, **dadurch gekennzeichnet, dass** die lösbare Kupplung ein wandlerseitiges Kupplungselement (22, 54, 58) und ein mikromanipulatorseitiges Kupplungselement (21, 45, 50, 60, 62, 68) aufweist, die wahlweise in und außer mechanischen Eingriff miteinander bringbar sind.

3. Hörsystem nach Anspruch 2, **dadurch gekennzeichnet, dass** das wandlerseitige Kupplungselement (22, 54, 58) zur festen Verbindung mit dem Wandler (12) bereits bei der Produktion des Wandlers oder erst im Zuge der Implantation des Wandlers ausgebildet ist.

4. Hörsystem nach einem der vorhergehenden Ansprüche, **dadurch gekennzeichnet, dass** das mikromanipulatorseitige Kupplungselement (21, 45, 60, 62, 68) eine Aufnahme (23, 44, 61, 74) für das wandlerseitiges Kupplungselement (22, 54, 58) bildet.

5. Hörsystem nach einem der vorhergehenden Ansprüche **dadurch gekennzeichnet, dass** mindestens eines der beiden Kupplungselemente (22, 45, 62, 68) mindestens partiell aus elastischem Werkstoff, insbesondere einem weichen Polymer, vorzugsweise einem Silicon, Polytetrafluorethylen oder Polyurethan, gefertigt ist.

6. Hörsystem nach einem der Ansprüche 1 bis 4, **dadurch gekennzeichnet, dass** beide Kupplungselemente (22, 45, 62, 68) aus nichtelastischem Werkstoff, insbesondere einem harten Polymer, vorzugsweise einem Polycarbonat, einem biokompatiblen Metall oder einem keramischen Werkstoff, gefertigt sind.

7. Hörsystem nach Anspruch 5, **dadurch gekennzeichnet, dass** die lösbare Kupplung (21, 45; 22) als Rastkupplung ausgebildet ist.

8. Hörsystem nach Anspruch 7, **dadurch gekennzeichnet, dass** das mikromanipulatorseitige Kupplungselement (21) als starre ringförmige Aufnahme ausgebildet ist sowie dass das wandlerseitiges Kupplungselement (22) mindestens partiell elastisch und in die starre ringförmige Aufnahme in Axialrichtung einrastbar ist.

9. Hörsystem nach Anspruch 7, **dadurch gekennzeichnet, dass** das mikromanipulatorseitige Kupplungselement (45) als aufspreizbare Gabel ausgebildet ist, in welche das wandlerseitiges Kupplungselement (22) in Radialrichtung einrastbar ist.

10. Hörsystem nach einem der Ansprüche 1 bis 6, **dadurch gekennzeichnet, dass** das mikromanipulatorseitige Kupplungselement (62) eine aufspreizbare Aufnahme (61) bildet, in die das wandlerseitige Kupplungselement (22) in Axialrichtung einführbar ist, und die in einer das wandlerseitige Kupplungselement (22) festhaltenden Stellung verrastbar ist.

11. Hörsystem nach einem der Ansprüche 1 bis 6, **dadurch gekennzeichnet, dass** das mikromanipulatorseitige Kupplungselement (68) als aufspreizbare Zange ausgebildet ist, in welche das wandlerseitige Kupplungselement (22) in Axialrichtung einführbar ist.

12. Hörsystem nach Anspruch 11, **dadurch gekennzeichnet, dass** die aufspreizbare Zange (68) in einer das wandlerseitige Kupplungselement (22) festhaltenden Schließstellung verriegelbar ist, vorzugsweise mittels einer entlang einem Teil der Zange verschiebbaren Schiebehülse (75).

13. Hörsystem nach einem der Ansprüche 1 bis 6, **dadurch gekennzeichnet, dass** die lösbare Kupplung (50, 60; 54, 58) als Steckkupplung mit zwei Kupplungselementen ausgebildet ist, von denen das eine in das andere einsteckbar ist und die im montierten Zustand über eine Presspassung miteinander in Eingriff gehalten sind.

14. Hörsystem nach Anspruch 13, **dadurch gekennzeichnet, dass** das eine Kupplungselement (50, 58) einen schwalbenschwanzförmigen Teil (51, 59) aufweist, der in eine komplementäre Aufnahmenut (52) des anderen Kupplungselements (54, 60) einschiebbar ist.

15. Hörsystem nach einem der Ansprüche 2 bis 14, **dadurch gekennzeichnet, dass** mindestens eines (21, 22) der beiden Kupplungselemente rotationssymmetrisch ausgebildet ist.

16. Hörsystem nach einem der Ansprüche 2 bis 15, **dadurch gekennzeichnet, dass** das mikromanipulatorseitige Kupplungselement (21, 45, 50, 60, 68) spiegelsymmetrisch mit Bezug auf die Wandlerachse ausgebildet ist.
